Europäisches Patentamt

(19) European Patent Office

Office européen des brevets

(11) Publication number: **0 326 419**
**A2**

## EUROPEAN PATENT APPLICATION

(21) Application number: 89300829.2

(22) Date of filing: 27.01.89

(51) Int. Cl.⁴: **C 12 N 15/00**
C 12 N 1/20, C 12 N 1/18,
C 12 N 5/00, A 61 K 37/547,
C 12 N 9/64

(30) Priority: 27.01.88 US 148749

(43) Date of publication of application:
02.08.89 Bulletin 89/31

(84) Designated Contracting States:
AT BE CH DE ES FR GB GR IT LI LU NL SE

(71) Applicant: THE UNITED STATES OF AMERICA as
represented by THE SECRETARY OF AGRICULTURE
United States Department of Agriculture
Washington, DC 20250 (US)

CODON
213 East Grand Avenue
South San Fransisco CA 94080 (US)

(72) Inventor: Pruett, John H. ,Jr.
212 Stephanie
Herrville Texas 87028 (US)

Files, James G.
1911 Lyon Avenue
Belmont California 94002 (US)

Kuhn, Irene
24A Cumberland Street
San Francisco California 78257 (US)

Temeyer, Kevin B.
25115, Danna Marie Drive
San Antonio Texas 78257 (US)

(74) Representative: Thomson, Paul Anthony
Potts, Kerr & Co. 15, Hamilton Square
Birkenhead Merseyside L41 6BR (GB)

The microorganism(s) has (have) been deposited with American Type Culture Collection under number(s) ATCC 67612, 67613.

(54) **Vaccines for the protection of animals against hypodermosis.**

㊸ This invention relates to the development of a vaccine against hypodermosis, a disease resulting from a maggot invasion by an insect taxonomically classified within the genus Hypoderma. The effective ingredients of the disclosed vaccines are hypodermins A, B, and C. Hypodermins A, B and C are serine proteases. Hypodermin C is generally known as collagenase. The hypodermins are produced naturally by the larvae of the insect. Methods for producing the hypodermins by recombinant genetics are disclosed as well as vaccines containing pure hypodermins and selected mixtures. Methods for immunoprotecting animals from Hypoderma species, especially Hypoderma lineatum are also provided. The precursor sequence of Hypodermin A and B is also disclosed for use as a general purpose signal sequence for the secretion of heterologous proteins expressed by recombinant insect cells.

FIG._I.

## Description

## VACCINES FOR THE PROTECTION OF ANIMALS AGAINST HYPODERMOSIS

## BACKGROUND OF THE INVENTION

### Field of the Invention

This invention relates to the development of a vaccine against hypodermosis, a disease resulting from a maggot invasion by an insect taxonomically classified within the genus Hypoderma. The effective ingredients of the disclosed vaccines are hypodermins A, B, and C. Hypodermins A, B and C are serine proteases. Hypodermin C is generally known as collagenase. The hypodermins are produced naturally by the larvae of the insect. Methods for producing the hypodermins by recombinant genetics are disclosed as well as vaccines containing pure hypodermins and selected mixtures. Methods for immunoprotecting animals from Hypoderma species, especially Hypoderma lineatum are also provided. The precursor sequence of Hypodermin A and B is also disclosed for use as a general purpose signal sequence for the secretion of heterologous proteins expressed by recombinant insect cells.

### Background

Hypodermosis is a maggot invasion of the connective tissues of animals. The disease is of economic significance to the cattle industry. Cattle contract the disease during the summer when female flies deposit eggs on the hair of the animals. The eggs hatch and the larva penetrate the hide. Migrating via connective tissue, the larvae eventually reach the back of the animal. Here, they digest a breathing hole in the hide and form a cavity called a "warble" where they reside for 45-60 days. Residence in the warble accounts for significant hide damage and intense inflammation in the area of dead or dying larvae. This inflammation impacts on general animal health. Warbles foul meat and account for significant losses during butchering as a result of additional meat trimming in the packing house.

The larvae eventually emerge from the warble. After falling to the ground, the larvae pupate and emerge as adult flys ready to continue the life cycle.

All breeds and sexes of cattle are susceptible to this disease, which affects cattle throughout the temperate northern zone. Current practice of control includes dipping and spraying with organophosphates. The organophosphate insecticides which are used against Hypoderma are systemic and will kill migrating larvae. However, because of the potential for hypersensitivity (anaphylactic) reactions, they are not recommended during those times of the year when the H. lineatum larvae are in the esophagus where localized sensitivity caused by killed larvae may produce vomiting, swallowing difficulties or respiratory problems and when H. bovis larvae are in the spinal column where localized sensitivity caused by killed larvae can lead to possible paralysis.

Two species of Hypoderma are of economic significance. They are Hypoderma lineatum and Hypoderma bovis.

### Information Disclosure

A succinct review of hypodermosis can be found in R. Jensen and D.R. Mackey, Diseases of Feedlot Cattle, 3rd Ed., Lea and Febiger, Philadelphia pp. 228-234. The development of acquired immunological resistance to infection with Hypoderma lineatum in cattle has been established, although such resistance is not completely effective in prevention of hypodermosis. Pruett, J.H. and Barrett, C.C., Kinetic Development of Humoral Anti-Hypoderma Lineatum Antibody Activity in the Serum of Vaccinated and Infested Cattle, Southwest. Entomol., 10:39-48 (1985).

Crude protein fractions of Hypoderma lineatum larval homogenates have been demonstrated to induce a hypersensitive skin reaction in cattle. Pruett, J.H. and Barrett, C.C., Induction of Intradermal Skin Reactions in the Bovine by Fractionated Proteins of Hypoderma Lineatum, Vet. Parasit., 16:137-146 (1981). Partially purified hypodermin C has been used as a vaccine in cattle. Maget A. and Boulard, C., "Study of the Immunization of Hypodermis of Cattle With a Vaccine Containing a Crude Extract of Collegenase From First Stage of H. Lineatum," C.R. Acad. Sci., Paris, 270:728-730 (1970). The kinetics of anti-hypoderma antibody production have been studied using crude protein extracts. Pruett, J.H. and Barrett, C.C., Southwest. Entomol., 10:39-48 (1985).

## SUMMARY OF THE INVENTION

The present invention provides segments of DNA comprising a cDNA sequence coding for a protein selected from the group consisting of hypodermins A, B, and C, immunogenic equivalents, and combinations thereof. These segments are described as being a part of plasmids which can be used for replication alone or for both replication and expression of the cDNA sequence when inserted into a suitable host. The host may be selected from among the group consisting of bacteria, yeast, and eukaryote cell cultures.

There is also described herein, suitable hosts containing a plasmid as described above. Said hosts may either replicate the disclosed plasmids or express the protein selected from the group consisting of hypodermins A, B, and C which by definition includes immunogenic equivalents, and combinations thereof.

Vaccines are also described herein for protection of animals from hypoderma species consisting of injectables of non-hypoderma origins and immunologically pure Hypoderma protein selected from the group consisting of:

    a. hypodermin A;
    b. hypodermin B;
    c. substantially pure hypodermin C;

    d. a mixture of hypodermin B and hypodermin C;
    e. a mixture of hypodermin A and hypodermin B; and
    f. a mixture of substantially pure hypodermin A, hypodermin B, and hypodermin C. The vaccines are preferably used to protect animals against Hypoderma species lineatum and bovis. These vaccines include those containing immunologically pure hypodermin A, and trace amounts of hypodermin B and hypodermin C. A preferred vaccine contains immunologically pure hypodermin A, B or C. Other vaccines disclosed herein comprise a mixture of hypodermin B and hypodermin C, thereof without trace amounts of hypodermin A, and vaccines wherein the mixture of hypodermin B and hypodermin C is substantially pure.

Methods are also disclosed for protecting animals from infection of Hypoderma species which comprises the vaccination of the animals with a vaccine consisting of injectables of non-hypoderma origins and essentially pure Hypoderma protein selected from the groups as described above.

Finally, a plasmid is described comprising a nucleotide sequence encoding the secretion signal peptide of hypodermin A or B, inclusive of natural polymorphism and synthetically created derivatives having equivalent ability to transmit proteins from inside an insect cell to the culture medium. A plasmid comprising a nucleic acid sequence encoding an insect secretion signal peptide, wherein the peptide is:
met-leu-lys-phe-val-ile-leu-leu-cys-ser-ile-ala-tyr-val-phe-gly-   or   met-leu-lys-phe-val-ile-leu-val-cys-ser-val-ala-cys-val-phe-gly-

Deposits of E. coli containing plasmids having cDNA encoding hypodermin A and hypodermin B have been made in accordance with the Budapest Treaty. E. coli culture having Accession No. 67,612 and containing cDNA encoding hypodermin A was deposited with the American Type Culture Collection (ATCC) at 12301 Parklawn Dr., Rockville, Maryland, U.S.A. on January 21, 1988 and E. coli culture having Accession No. 67,613 and containing cDNA encoding hypodermin B was deposited with the ATCC on January 21, 1988. Both clones contain the pUC-18 plasmid which is commercially available. The hypodermin sequences have been inserted into the EcoRI site of the polylinker.

BRIEF DESCRIPTION OF THE FIGURES

    Fig. 1. Sephadex DEAE-A50 ion exchange elution profile of Hypodermin lineatum 1st instar gullet larval antigens.
    Fig. 2. E. coli expression plasmid construction summary.
    Fig. 3. Mammalian cell expression plasmid construction summary.
    Fig. 4. Insect cell (baculovirus) expression plasmid construction.

DETAILED DESCRIPTION OF THE PREFERRED EMBODIMENT

`The natural levels of resistance in previously infested bovine are sufficient to substantially reduce the total fly population to levels of economic insignificance. However, it is from naive bovine and from those susceptible bovine which never develop significant resistance to Hypoderma that significant larvae emerge to maintain and increase the fly population. It is the objective of the disclosed vaccines to increase bovine resistance in naive and susceptible animals to levels that both minimize physical damage to the animals and prevent said animals from contributing substantively to the endogenous fly population.

This invention relates to the use of purified hypodermins A, B, and C for the immunoprotection of agriculturally important animals, especially bovine cattle. The term "cattle" is meant to include both the dairy and meat producing animals. The term "hypodermins" is meant to include the naturally occurring serine proteases and collagenases produced by the larva of Hypoderma spp. They can be isolated and purified directly from these insects as described below.

By naturally-occurring, it is understood that polymorphic species of protein probably exist in sub-populations of Hypodermin spp., i.e., minor differences in the primary amino acid sequences of naturally-occurring hypodermins from H. bovis and H. lineatum might be discovered. The term "hypodermins" includes these variant proteins.

To facilitate the production of the hypodermins, one can use recombinant genetics to introduce a gene encoding one of the hypodermins into a suitable host cell and then induce that cell to produce large amounts of the selected hypodermin protein. This invention embraces molecular genetic manipulations that can be achieved in a variety of known ways. The following descriptions will detail the various methods available to express genes encoding hypodermins A, B, and C, and is followed by specific examples of preferred methods.

In summary, the manipulation can be described as the obtaining of a cDNA of a hypodermin, the cloning and replication of the cDNA in E. coli and the expression of the cDNA in a suitable host. The desired recombinant product is then recovered from the host cells and formulated into a vaccine.

A. General Methods

Generally, the nomenclature and general laboratory procedures required in this application can be found in Maniatis, T. et al., Molecular Cloning - A Laboratory Manual, Cold Spring Harbor Laboratory, Cold Spring Harbor, New York, 1985. The manual is hereinafter referred to as "Maniatis".

All E. coli strains are grown in Luria broth (LB) and M9 medium supplemented with glucose and acid-hydrolyzed casein amino acids. Strains with resistance to antibiotics are maintained at the drug concentrations described in Maniatis.

All enzymes are used according to the manufacturer's instructions.

Libraries are constructed in bacteriophage λ vectors. Phage are packaged in vitro, as described in Maniatis. Recombinant phage are analyzed by plaque hybridization, as described in Benton and Davis, Science, 196:180-182 (1977), and in Maniatis.

Plasmids are sequenced using the modified dideoxy procedure of Tabor and Richardson, P.N.A.S., 84:4767-4771 (1987). Sequencing is performed with a DNA sequencing kit from U.S. Biochemicals according to the manufacturer's specifications.

Polynucleotide sizes are given in either kilo-bases (kb) or basepairs (bp). These are estimates derived from agarose gel electrophoresis.

B. Hypodermin cDNA

The first step in obtaining expression of genes encoding hypodermins A, B, and C is to obtain the DNA sequence coding for the protein from cDNA clones. This sequence is then cloned into a plasmid which is capable of directing transcription of the gene and allowing efficient translation of the transcript.

The method for obtaining cDNA encoding the hypodermin proteins has been described generally in Maniatis. A library of clones is prepared by adding EcoRI linkers to the cDNA, ligating the cDNA to EcoRI digested DNA arms of λ gt10 and packaging the ligated DNA into viable phage particles. The clones containing full length hypodermin cDNA are detected by hybridization with probes of labeled synthetic oligonucleotides complementary to portions of the sequences, followed by restriction enzyme analysis and DNA sequencing. Wallace, R.B., Johnson, N.J., Hirose T., Miyake M., Kawashima, E.H., and Itakura, K. (1981), Nucleic Acids Res. 9:879.

The synthetic oligonucleotide probes are single-strand DNA molecules, typically between 10 and 50 nucleotides in length. Their sequence is based on the amino acid sequence of the protein encoded by the gene of interest. Because the genetic code is degenerate, i.e., from 1 to 6 codons can specify a single amino acid, a unique DNA sequence cannot be deduced. In practice, a region of protein sequence is chosen which contains amino acids of minimum codon degeneracy. Then a mixture of all possible DNA sequences encoding this protein sequence is synthesized. Oligonucleotides for use as probes are chemically synthesized according to the solid phase phosphoramidite triester method first described by Beaucage, S.L. and Caruthers, M.H., Tetrahedron Letts., 22(20):1859-1862 (1981) using an automated synthesizer, as described in Needham-VanDevanter, D.R., et al., Nucleic Acids Res., 12:6159-6168 (1984). Purification of oligonucleotides is by either native acrylamide gel electrophoresis or by anion-exchange HPLC as described in Pearson, J.D. and Regnier, F.E., J. Chrom., 255:137-149 (1983).

The sequence of the synthetic oligonucleotides can be verified using the chemical degradation method of Maxam, A.M. and Gilbert, W., Grossman, L. and Moldave, D., eds. Academic Press, New York, Methods in Enzymology, 65:499-560 (1980).

C. Expression in E. coli

To obtain high level expression of a cloned gene, such as those cDNAS encoding the hypodermin proteins A. B. and C in a prokaryotic system, it is essential to construct expression plasmids which contain, at the minimum, a strong promoter to direct mRNA transcription, a ribosome binding site for translational initiation, and a transcription terminator. Examples of regulatory regions suitable for this purpose are the E. coli beta-galactosidase promoter of the lac operon as described in Experiments in Molecular Biology, Miller, J.H., ed. Cold Spring Harbor, New York (1972); the promoter and operator region of the E. coli tryptophan biosynthetic pathway as described by Yanofsky, C., Kelley, R.L. and Horn, V., J. Bacteriol., 158:1018-1024 (1984); the leftward promoter of phage lambda (PL) as described by Herskowitz, I. and Hagen, D., Ann. Rev. Genet., 14:399-445 (1980); and the tac and trc synthetic promoters derived from lac and trp described by Brosius, J., et al., J. Biol. Chem., 260:3539-3541 (1985) and DeBoer, H.A., et al., P.N.A.S., 80:21-25 (1983).

A hypodermin protein produced in E. coli may not necessarily fold properly. During purification from E. coli, the expressed hypodermin proteins may first be denatured and then renatured. This can be accomplished by solubilizing the E. coli produced proteins in a chaotropic agent such as guanidine HCl or urea and reducing all the cysteine residues with a reducing agent such as beta-mercaptoethanol or dithiothreitol. The protein is then renatured by dilution and air oxidation, by slow dialysis, or by gel filtration (U.S. Patent No. 4,511,503).

The protein can also renature in the presence of reduced and oxidized glutathione. See, e.g., R.R. Hantgan, et al., Biochemistry, 13:3421-3431 (1974); Saxena, V.P., and Watlaufer, D.R., Biochemistry, 9:5015 (1970); Ahmed, A.K., et al., J. Biol. Chem., 250:8477-8482 (1975).

Finally, one may renature the protein by using a disulfide exchange to produce an S-sulfonate derivative. See, Wetzel, R., et al., Gene, 16:3-71 (1981) and Bailey and Cole, J. Biol. Chem., 234:1733-1734 (1959) for making the S-sulfonate derivative.

Renaturation should be performed in the presence of protease inhibitors to prevent self digestion of the renaturing protease. Epstein, C.J. and Anfinsen, C.B., J. Biol. Chem., 237:3464-3467 (1962).

Detection of the hypodermin proteins is achieved by methods known in the art such as radioimmunoassays, or Western blotting techniques or immunoprecipitation. Purification from E. coli can be achieved following procedures described in U.S. Patent No. 4,511,503.

D. Synthesis of the Hypodermin Proteins in Yeast

Synthesis of heterologous proteins in yeast is well known and described. Methods in Yeast Genetics, Sherman, F., et al., Cold Spring Harbor Laboratory, (1982) is a well recognized work describing the various methods available to produce hypodermin proteins in yeast.

For high level expression of a gene in yeast, it is essential to connect the gene to a strong promoter system as in the prokaryote and to also provide efficient transcription termination/polyadenylation sequences from a yeast gene. Examples of useful promoters include GAL1,10 (Johnson, M., and Davies, R.W., Mol. and Cell. Biol., 4:1440-4S, 1984), ADH2 (Russell, D., et al., J. Biol. Chem., 258:2674-2682, 1983), PHO5 (EMBOJ. 6:675-680, 1982), and MFα1. A multicopy plasmid with a selective marker such as Leu-2, URA-3, Trp-1, and His-3 is also desirable.

The MFα1 promoter is preferred. The MFα1 promoter in a host of the α mating-type is constitutive, but is off in diploids or cells with the a mating-type. It can, however, be regulated in cells having a ts mutation at one of the SIR loci by raising or lowering the temperature at which the cells are grown. The effect of such a mutation on α type cells grown at 35° C is to turn on the normally silent gene coding for the a mating-type. The expression of the a mating-type gene, in turn, turns off the MFα1 promoter. Lowering the temperature of growth to 27° C reverses the whole process, i.e., turns off the a mating-type and turns on the MFα1. Herskowitz, I. and Oshima, Y. in The Molecular Biology of the Yeast Saccharomyces, Strathern, J.N. et al., eds., Cold Spring Harbor Lab., Cold Spring Harbor, N.Y., pp. 181-209 (1982).

The polyadenylation sequences are provided by the 3'-end sequences of any of the highly expressed genes, like ADH1, MFα1, or TPI (Alber, T. and Kawasaki, G., J. of Mol. & APPI. Genet. 1:419-434, 1982).

A number of yeast expression plasmids like YEp6, YEp13, YEp24 can be used as vectors. A gene of interest such as hypodermin A, B, or C cDNA can be fused to any of the promoters in various yeast hosts. The above-mentioned plasmids have been fully described in the literature (Borstein, et al., Gene, 8:17-24, 1979; Broach, et al., Gene, 8:121-133, 1979).

Two procedures are used in transforming yeast cells. In one case, yeast cells are first converted into protoplasts using zymolyase, lyticase or glusulase, followed by addition of DNA and polyethylene glycol (PEG). The PEG-treated protoplasts are then regenerated in a 3% agar medium under selective conditions. Details of this procedure are given in the papers by J.D. Beggs, Nature (London), 275:104-109 (1978); and Hinnen, A., et al., Proc. Natl. Acad. Sci. USA, 75:1929-1933 (1978). The second procedure does not involve removal of the cell wall. Instead the cells are treated with lithium-chloride or acetate and PEG and put on selective plates (Ito, H., et al., J. Bact., 153:163-168, 1983).

The hypodermin proteins can be isolated from yeast by lysing the cells and applying standard protein isolation techniques to the lysates. The procedures developed for the hypodermin A from H. lineatum larva are illustrative. The monitoring of the purification process can be accomplished by using Western blot techniques or radioimmunoassays.

E. Expression in Cell Cultures

The hypodermin cDNA can be ligated to various expression vectors for use in transforming host cell cultures. The vectors typically contain gene sequences to initiate transcription and translation of the hypodermin gene. These sequences are generally compatible with the selected host cell.

In addition, the vectors preferably contain a marker to provide a phenotypic trait for selection of transformed host cells such as dihydrofolate reductase, neomycin resistance, hygromycin resistance, or metallothionein. The enzyme beta-galactosidase and the nuclear polyhedral virus from Autographa californica are useful for insect cells. Additionally, a replicating vector might contain a replicative origin.

Illustrative of cell cultures useful for the production of the hypodermin proteins are cells of insect or mammalian origin. Mammalian and insect cell systems often will be in the form of monolayers of cells although mammalian cell suspensions may also be used. Illustrative examples of mammalian cell lines include VERO and HeLa cells, Chinese hamster ovary (CHO) cell lines, WI38, BHK, COS-7, Bowes melanoma, or MDCK cell lines. Examples of insect cell lines are Spodoptera frugiperda Sf9 cells (fall armyworm), Bombyx mori, and Drosophila melanogaster embryonic cell line S2 (or Schneider cells).

As indicated above, the vector, e.g., a plasmid, which is used to transform the host cell, preferably contains gene sequences to initiate the transcription and sequences to control the translation of the hypodermin protein gene sequence. These sequences are referred to as expression control sequences. When the host cell is of insect or mammalian origin illustrative expression control sequences are obtained from the SV-40 promoter (Science, 222:524-527, 1983), the CMV I.E. promoter (Proc. Natl. Acad. Sci. 81:659-663, 1984) or the metallothionein promoter (Nature 296:39-42, 1982). The plasmid or replicating or integrating DNA containing the expression control sequences is cleaved using restriction enzymes and adjusted in size as necessary or desirable and ligated with cDNA coding for a hypodermin protein by means well known in the art.

As with yeast, when higher animal host cells are employed polyadenlyation or transcription terminator sequences from known mammalian genes need to be incorporated into the vector. An example of a terminator sequence is the polyadenlyation sequence from the bovine growth hormone gene.

Additionally gene sequences to control replication in the host cell may be incorporated into the vector such as those found in bovine papillomavirus type-vectors. Saveria-Campo, M., Bovine Papillomavirus DNA: a Eukaryotic Cloning Vector" in DNA Cloning Vol. II a Practical Approach Ed. D.M. Glover, IRL Press, Arlington, Virginia pp. 213-238 (1985).

The host cells are competent or rendered competent for transformation by various means. There are several well-known methods of introducing DNA into animal cells. These include: calcium phosphate precipitation, fusion of the recipient cells with bacterial protoplasts containing the DNA, treatment of the recipient cells with liposomes containing the DNA, and micro-injection of the DNA directly into the cells.

The transformed cells are cultured by means well known in the art. Biochemical Methods in Cell Culture and Virology, Kuchler, R.J., Dowden, Hutchinson and Ross, Inc., (1977). The expressed hypodermin is isolated from cell suspensions created by disruption of the host cell system by well known mechanical or enzymatic means.

Isolation of the hypodermin protein can be accomplished by lysing the host cells with detergents. Further purification is accomplished in the manner described for the purification of the hypodermin from H. lineatum larva. Pruett and Barret, Vet. Paras., 16:137- 146 (1984) and in the examples provided below.

Hypodermin cDNA may alternatively be produced in the insect cell lines described above using the baculovirus system. This system has been described by Luckow, V.A. and Summers, M.D., Bio/Technology 6:47-55 (1988). Generally, this expression system provides for a level of expression higher than that provided by most mammalian systems. The baculovirus infects the host insect cells, replicates its genome through numerous cycles, and then produces large amounts of polyhedrin crystals. The polyhedrin gene can be replaced with a hypodermin cDNA gene. The polyhedrin promoter will then make large amounts of hypodermin protein following infection of the culture host cell and replication of the baculovirus genome. The non-secreted gene product is harvested from the host 3-7 days post infection. Alternatively, the hypodermin protein may be secreted from the cells if appropriate membrane signal sequences are present on the protein.

## F. Vaccines Against Hypoderma Spp

A vaccine prepared utilizing the hypodermins or immunogenic equivalents thereof can consist of: (a) fixed cells either recombinantly altered to produce hypodermin proteins or cells from the Hypoderma larva itself; (b) a crude cell extract; (c) a partially or completely purified hypodermin preparation. The hypodermin immunogens can be prepared in vaccine dose form by well-known procedures. The vaccine may be in the form of an injectable dose and may be administered intramuscularly, intravenously, or subcutaneously. The vaccine may also be administered intranasally by aspiration, or orally by mixing the active components with feed or water, providing a tablet form, and the like. Means for administering the vaccine should be apparent to those skilled in the art from the teachings herein; accordingly, the scope of the invention is not limited to a particular delivery form.

For parenteral administration, such as subcutaneous injection, the immunogen may be combined with a suitable carrier, for example, it may be administered in water, saline or buffered vehicles with or without various adjuvants or immunomodulating agents such as aluminum hydroxide, aluminum phosphate, aluminum potassium sulfate (alum), beryllium sulfate, silica, kaolin, carbon, water-in-oil emulsions, oil-in-water emulsions, muramyl dipeptide, bacterial endotoxin, lipid X, Corynebacterium parvum (Propionobacterium acnes), Bordetella pertussis, polyribonucleotides, sodium alginate, lanolin, lysolecithin, vitamin A, saponin, liposomes, levamisole, DEAE-dextran, blocked copolymers or other synthetic adjuvants. Such adjuvants are available commercially from various sources, for example, Merck Adjuvant 65 (Merck and Company, Inc., Rahway, N.J.) or Freund's Incomplete Adjuvant and Complete Adjuvant (Difco Laboratories, Detroit, Michigan). Other suitable adjuvants are Amphigen (oil-in-water), Alhydrogel (aluminum hydroxide), or a mixture of Amphigen and Alhydrogel.

The Rotavirus VP6 carrier system developed by VIDO (Veterinary Infectious Disease Organization, Saskatoon, Canada), although not an adjuvant, is also suitable.

The proportion of immunogen and adjuvant can be varied over a broad range so long as both are present in effective amounts. For example, aluminum hydroxide can be present in an amount of about 0.5% of the vaccine mixture ($Al_2O_3$ basis). On a per-dose basis, the amount of the immunogen can range from about 1.0 μg to about 100 mg per bovine host. A preferable range is from about 5 μg to about 2.0 mg per dose. A suitable dose size is about 1-10 ml, preferably about 1.0 ml. Accordingly, a dose for intramuscular injection, for example, would comprise 1 ml containing 1.0 mg of immunogen in admixture with 0.5% aluminum hydroxide. Comparable dose forms can also be prepared for parenteral administration to calves, but the amount of immunogen per dose will be smaller, for example, about 0.75 to about 1.5 mg per dose.

For the initial vaccination of immunologically naive cows, a regimen of between 1 and 4 doses can be used with the injections spaced out over a 2- to 6-week period. Typically, a two-dose regimen is used. The second dose of the vaccine then should be administered some weeks after the first dose, for example, about 2 to 4 weeks later. Animals that have been previously exposed to Hypoderma or have received colostral antibodies from the mother may require booster injections. The booster injection is preferably timed to coincide with the vulnerable point in the life cycle of the fly. For example, booster injections can be made 4 to 6 months into the life cycle, which corresponds to an injection, approximately one month before larvae migrate to the back of the animals. A two-dose regimen is considered preferable for the most effective immunization of the calves. Semiannual revaccination is recommended for breeding animals. Steer or bulls may be revaccinated at any time preferably shortly before adult flies are active. Also, cows can be revaccinated before breeding. Calves may be vaccinated at about 1-3 months after birth, again at 4 to 6 months, and yearly or preferably semiannually thereafter.

The vaccine may also be combined with other vaccines for other diseases to produce multivalent vaccines. It may also be combined with other medicaments, for example, antibiotics. A pharmaceutically effective amount of the vaccine can be employed with a pharmaceutically acceptable carrier such as viral capsid protein complex or diluent understood to be useful for the vaccination of animals such as swine, cattle, sheep, goats, and other mammals. These additives including adjuvants are referred to as "injectables of non- hypoderma origins."

Other vaccines may be prepared according to methods well-known to those skilled in the art as set forth, for example, in Tizard, I., An Introduction to Veterinary Immunology, 2nd Ed (1982), which is incorporated herein by reference.

## G. Definitions

The phrase "cell culture" refers to the containment of growing cells derived from either a multicellular plant or animal which allows for the cells to remain viable outside the original plant or animal.

The term "microorganism" includes both single cellular prokaryote and eukaryote organisms such as bacteria, actinomycetes and yeast.

The term "plasmid" refers to an autonomous self-replicating circular DNA and includes both the expression and nonexpression types. Where a recombinant microorganism or cell culture is described as hosting an "expression plasmid", this includes both extrachromosomal circular DNA and DNA that has been incorporated into the host chromosome(s). Where a plasmid is being maintained by a host cell, the plasmid is either being stably replicated by the cells during mitosis as an autonomous structure or is incorporated within the host's genome.

The term "promoter" is a region of DNA involved in binding the RNA polymerase to initiate transcription.

The term "hypodermin" when describing hypodermin A and B or hypodermin C (hypoderma collagenase) as separated components (e.g. hypodermin A) or as mixtures (e.g. hypodermin A, B or C) is meant to include both the naturally occurring proteins, derivative polypeptides embracing deletions and changes in the amino acid sequence such that they appear as functional equivalents to the immune system for purposes of protection from hypodermosis and combinations thereof. These non-natural derivatives are also known as "immunogenic equivalents". Those of skill will readily recognize that it is only necessary to expose a mammal to appropriate epitopes in order to elicit effective immunoprotection. The epitopes are typically segments of amino acids which are a small portion of the whole protein. Using recombinant genetics it is simple and routine to alter a natural protein's primary structural to create derivatives embracing epitopes that are identical to or substantially the same as (immunologically equivalent) the naturally occurring epitopes. Such derivatives may include peptide fragments, amino acid substitutions, amino acid deletions and amino acid additions within the natural amino acid sequence for one of the hypodermins. For example, it is known in the protein art that certain amino acid residues can be substituted with amino acids of similar size and polarity without an undue effect upon the biological activity of the protein. In the primary sequence of the hypodermins, the following residues are generally considered to be interchangeable: (1) alanine, leucine, isoleucine, valine and proline are interchangeable, (2) phenylalanine and tryptophan are interchangeable, (3) serine, theronine and tyrosine are interchangeable, (4) asparagine and glutamine are interchangeable, (5) lysine, arginine, histidine and ornithine are interchangeable, and (6) aspartic acid and glutamic acid.

The phrase "DNA sequence" refers to a single or double stranded DNA molecule comprised of nucleotide bases, adenosine, thymidine, cytosine and guanosine.

The phrase "immunologically pure," in the context of hypodermin protein, refers to compositions of protein containing hypodermin A, B, or C. Immunologically pure proteins may be contaminated with low levels of protein from the Hypoderma larvae or from recombinant host cell constituents. The level of contaminating protein is such that the vaccinated animal will not respond with significant levels of antibodies against said contaminants. Typically, the proteins will be pure to about at least 75% preferably at a purity in excess of 95% and most preferably in excess of 98%.

The phrase "substantially pure," in the context of hypodermin protein, refers to compositions of protein which are devoid of contaminating structural and non-structural protein produced by Hypodermin larvae. Such proteins are typically derived from genetically altered bacteria or eukaryote cells and may contain trace amounts of substances from those host cells.

The phrase "suitable host" refers to a cell culture or microorganism that is compatible with a recombinant plasmid and will permit the plasmid to replicate, to be incorporated into its genome or to be expressed.

The phrase "trace amounts" refers to concentration levels of contaminating protein in a vaccine preparation that are insufficient to elicit effective immunogenic protection when administered to a mammal.

## Example 1. Isolation of Hypodermin A from Larval Crude Antigens

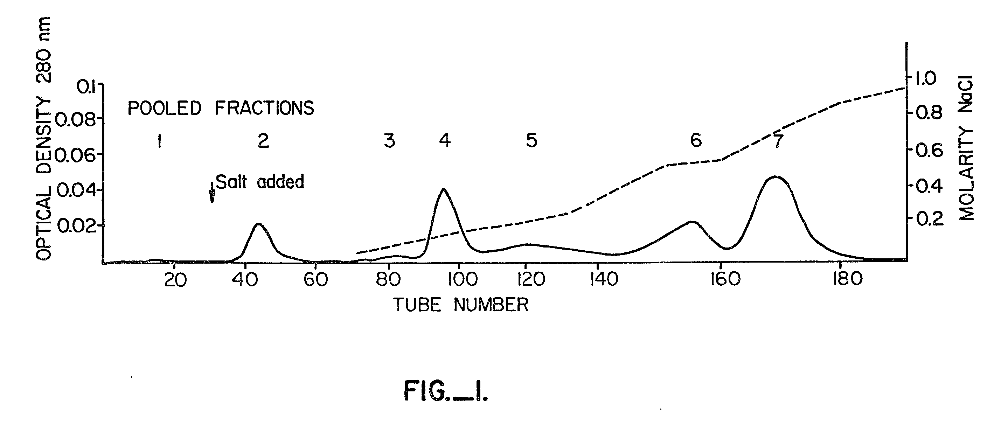

H. lineatum first-instar antigens are prepared by homogenizing larvae in a Tenbroeck tissue homogenizer with a 10mM Tris-HCl buffer, pH 7.4, 4°C. The homogenate is centrifuged at 48,000 x g, 4°C for 45 min (Sorval RC5B). Supernatant fluid is filtered through a sterilized 0.45 μm Swinnex filter (Millipore, Bedford, MA). Protein concentration is determined by a modified Lowry technique (Bradshaw, Introd. to Biological Tech., Prentice-Hall, Inc., Engle Cliffs, N.J., 107 pp. 1966). The crude antigens are stored at -20°C until needed. As described in J. Pruett and C. Barrett, Vet. Parasit., 16:137-146 (1984), DEAE-A50 (Pharmacia), an anion exchanger, is used to separate soluble proteins of H. lineatum in the antigen preparation. H. lineatum antigens (17.0 mg in 1.0 ml) are added to the column (2.6 x 23 cm) and eluted with a 0.01M Tris-HCl buffer, pH 7.5. A NaCl gradient (0-1.0M) is initiated after the fall- through peak eluted. The column is monitored by absorbance of 280 nm optical density $A_{280}$ (See Fig. 1). Fraction 4 is the first peak to elute from the column after the start of the gradient and contained at least three proteins of which hypodermin A is predominant. Proteins eluted in fraction 4 are concentrated by lyophilization, reconstituted in a minimum amount of distilled water and dialyzed against 10mM Tris-HCl, pH 7.4. Hypodermin A can be further purified by HPLC reverse phase chromatography (Vydac C4 column).

Hypodermins B and C can be isolated from the H. lineatum larval extracts by DEAE chromatography, as described in above. Fractions containing hypodermin B and C are run on HPLC reverse phase chromatography (Vydac C4 column) see Fig. 1. HPLC fractions containing predominantly hypodermin B are collected, concentrated by lyophilization, reconstituted in distilled water, and sequenced on an Applied Biosystems gas-phase protein sequencer.

## Example 2. Recombinant Clones Containing the Hypodermins A, B. and C cDNA

### A. Hypodermins A and B

The following procedure details the isolation of both hypodermin A and B cDNA using synthetic probes complementary to the N-terminal portions of the proteins. The amino acid sequences of hypodermin A and B are provided in Tables I and II, respectively.

Messenger RNA is isolated from first instar larvae of H. lineatum. Larvae are excised from esophagi of freshly slaughtered cattle and frozen directly in liquid nitrogen. RNA is extracted by a hot phenol/chloroform method, essentially as described by Jewett (Preparation of nucleic acids, Drosophila - A Practical Approach, Roberts. D.B., ed., pp. 275-286, IRL Press, Washington, D.C. (1986)). Frozen larvae of one gram or less are ground in a Tenbroeck tissue grinder (Wheaton) with 8 ml phenol (at 70°C, preequilibrated with 0.2M sodium acetate, pH 5.0), followed by addition and mixing of 0.4 ml 20% sodium dodecylsulfate (SDS) and 8 ml 0.2M sodium acetate, pH 5.0. The mixture is allowed to cool slightly, 8 ml chloroform is added and mixed, and the mixture is centrifuged to separate the phases. The upper aqueous layer containing the nucleic acids is removed. The organic phase is reextracted with 4 ml 0.2M sodium acetate, pH 5.0, and the aqueous extracts are pooled, reextracted with an equal volume of phenol- chloroform (1:1), and precipitated by the addition of 0.5 volumes of 7.5M ammonium acetate and 2.5 volumes of 100% ethanol. The precipitate is collected by centrifugation and dissolved in sterile glass distilled water. Messenger RNA is purified from the nucleic acid fraction by chromatography on oligo(dT)-cellulose (0.5 g, type 3, prepacked column, Collaborative Research, Inc., Bedford MA) according to the manufacturer's instructions. Messenger RNA is collected by ethanol precipitation and redissolved in sterile-glassed distilled water.

H. lineatum messenger RNA is converted into cDNA by the action of reverse transcriptase (Maniatis, et al., pp. 213-216). Approximately 8 μg of MRNA is used to synthesize cDNA using a cDNA synthesis system from Amersham, according to the manufacturer's instructions.

A recombinant library is made with this H. lineatum cDNA in bacteriophage α gt10 (Huynh, T.V., Young, R.A., and Davis, R. (1985), in DNA Cloning Techniques, A Practical Approach, Vol. 1, IRL Press, Oxford, D.M. Glover, ed., pp. 49-78). Approximately 2 μg of cDNA is methylated with EcoRI methylase (New England Biolabs) to block any EcoRI restriction sites. The synthetic DNA linker GGAATTCC, containing the EcoRI restriction sequence, is phosphorylated with polynucleotide kinase and ligated to the cDNA with T4 DNA ligase. This material is digested with EcoRI, resulting in cDNA with EcoRI cohesive ends. This cDNA is separated from unattached EcoRI linkers by chromatography on Sephacryl S-500 (Pharmacia). The EcoRI linker-cDNA is next ligated to ECORI-cleaved, dephosphorylated λ gt10 arms (Stratagene). Ligated λ gt10-cDNA is packaged into λ phage particles using "Gigapack Plus" packaging extracts (Stratagene), according to the manufacturer's instructions. These phage constitute the H. lineatum cDNA library.

The design and synthesis of oligonucleotide probes for screening the H. lineatum cDNA library require amino acid sequence information. Hypodermin A protein is isolated as described in Example 1. 100 μg of purified antigen is dialyzed against 50 mM Tris-HCl and sequenced on an Applied Biosystems gas-phase protein sequencer. The amino terminal sequence obtained is:

ile-val-gly-gly-val-glu-ser-lys-ile-glu-asp-phe-pro-trp-gln-ile-ser-leu-gln-arg-asp-gly-arg-his-tyr-xaa-gly-gly-ser-ile-tyr-ser-lys-asn-val-ile-ile-thr-ala-ala-

This sequence contains changes from the published hypodermin A sequence (Tong, et al. (1981), Biochim. Biophys. Acta 658, 209-219) at four of the first 21 residues. Based on this sequence, the following synthetic oligonucleotide mixture is designed to encode amino acids 10-16 (which contain amino acids of minimum codon degeneracy) and synthesized on a Biosearch DNA synthesizer. The mixture of 64 different 20-mers is:

```
GAA GAT TTT CCG TGG CAA AT
  G   C   C   A       G
              T
              C
```

There is conservation of some amino acid sequences between hypodermins A and B. The sequence of residues 10-16 of hypodermin B is:
glu-asp-phe-pro-trp-gln-val
Residues 10-15 of this sequence are identical to hypodermin A. (The previously published sequence has a glutamic acid residue at position 15. Eur. J. Biochem. 134: 261-267, (1983). Thus, the oligonucleotide mixture that encodes residues 10-16 of hypodermin A will have sufficient sequence homology with the gene encoding hypodermin B to identify clones of hypodermin B, as well as of hypodermin A, upon library screening.

To distinguish between clones of hypodermin A and hypodermin B, positive phage are probed with the following mixture based upon the N-terminal amino acids 33-39:

```
AAA AAT GTG ATT ATT ACG GC
  G   C   A   C   C   A
          T   A   A   T
          C           C
```

The λ gt10-H lineatum cDNA library is screened by the plaque hybridization method of Benton and Davis (Science, 196, 180-182, 1977), following Maniatis, et al. (pp. 320-321), using $^{32}$P labeled synthetic oligonucleotide probes. The library is plated on 160 mm NZCYM agar plates in NCZYM top agar (Maniatis, et al.) at $10^4$ pfu/plate (10,000 plaque forming units per plate) and incubated at 37°C for 10-15 hours. Filter replicas of the plates are taken with nitrocellulose filters (BA85, Schleicher and Schuell), denatured, fixed, and hybridized at $^{32}$P labeled oligonucleotide probes according to Maniatis, et al.

Positive λ gt10 phage clones are picked and replated and rescreened to assure that each comes from a single probe-positive phage. Purified phage are prepared from several positive clones, and the recombinant phage DNA is isolated and the cDNA insert subcloned into pUC18 (See Example 3, plasmid #1). Subclone DNA is prepared and subjected to DNA sequence analysis in order to determine the sequence of the ends of the cDNA inserts. DNA sequences corresponding to the 5′ end of the mRNA are compared to the N-terminal amino acid sequences to identify and to distinguish between clones encoding hypodermin A and hypodermin B.

B. Hypodermin C
The amino acid sequence of Hypodermin C is reported in Lecroisey, et al. (1987), J. Biol. Chem. 262, 7546-7511. The following synthetic oligonucleotides, designed from hypodermin C amino acid sequences, are synthesized and used as hybridization probes to identify clones encoding hypodermin C. Probe 3 is the primary probe for library screening.
1. Amino Acids 218-226:

```
Met-Asp-Trp-Ile-Gln-Gln-Asn-Thr

ATG GAT TGG ATA CAA CAA AAT AC
      C       T   G   G   C
              C
```

A mixture of 48 23-mers
2. Amino Acids 79-85 :

```
Met-Phe-Asn-Pro-Asp-Thr

ATG TTT AAT CCG GAT AC
        C   C   A   C
                T
                C
```

A mixture of 32 17-mers
   3. Amino Acids 35-40 :

```
Ile-Asp-Asn-Lys-Trp-Ile

ATT GAT AAT AAA TGG AT
  C   C   C   G
  A
```

A mixture of 24 17-mers. The library of Example 2B is screened as in that of Example 2A.

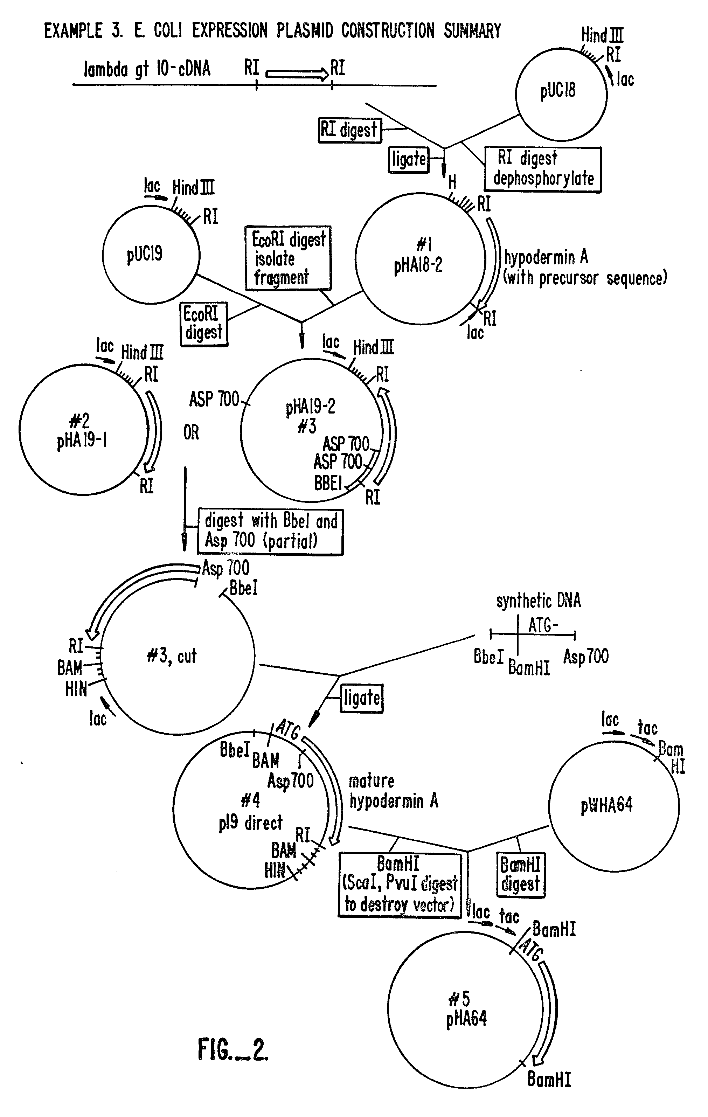

Example 3. Production of Hypodermin A in Bacterial Cells Construction of E. coli expression plasmid (FIG. 2)

DNA of λ gt10 clones encoding hypodermin A is digested with EcoRI restriction endonuclease to excise the cDNA insert from the phage vector. The cDNA insert is ligated into dephosphorylated (See Maniatis p. 133) plasmid vector pUC18 that has been previously cut with Eco-RI. A recombinant plasmid (plasmid #1) is isolated with the cDNA insert oriented with the pUC18 polylinker upstream from the direction of reading of the hypodermin gene, as determined by restriction mapping and DNA sequencing of the plasmid.

The mature hypodermin A protein is most likely the product of post-translational processing of precursor protein. Synthesis of the mature protein in E. coli requires removal of that portion of the gene encoding protein that would be removed during processing in vivo in H. lineatum. To accomplish this, purified DNA from the plasmid (plasmid #1) is digested with the restriction endonuclease EcoRI, the RI-fragment encoding hypodermin precursor is isolated by gel electrophoresis and ligated into EcoRI cut plasmid vector pUC19. Recombinant plasmids with the cDNA oriented in both directions are isolated. Plasmid #2 comprises cDNA inserts whose orientation is consistent with the direction of reading of the lac promoter, plasmid #3 comprises inserts whose orientation is counter to the direction of reading of the lac promoter. Plasmid #3 is digested to completion with Bbel and partially digested with Asp700. Synthetic oligonucleotides COD 872 and COD 873 are synthesized and when annealed, comprise a linker which contains 1) the cohesive end of the Bbel restriction site, 2) a BamHI site, 3) an ATG codon, and 4) sequences encoding the first 10 amino acids of the mature hypodermin A protein (i.e., ile-val-gly-gly, etc.). This double-stranded synthetic fragment (COD 872 plus COD 873) encoding the N- terminus of the mature protein is then ligated into plasmid #3 containing the C-terminal portion of the hypodermin A gene such that all the codons of the precursor protein removed by the Asp700 digestion are replaced (Table III). This ligation product is used to transform E. coli, and the new recombinant plasmid (plasmid #4) is isolated and its DNA purified. Identity of this clone is confirmed by DNA sequence analysis of the newly synthesized and inserted sequences.

Plasmid #4 is digested with BamHI and the modified hypodermin cDNA insert is purified and ligated into the BamHI site of direct expression vector pWHA64. Transformants from this ligation are screened for a plasmid with cDNA inserted in the same reading orientation as the vector expression signals. The resulting expression plasmid (plasmid #5) was transformed into E. coli strains SG936, CAG2039, DH5α, M3110 and MTM6/pFSI$^Q$.

To synthesize hypodermin A in E. coli, cultures of SG936 carrying the expression plasmid are grown in rich medium (e.g., L broth), containing ampicillin to maintain the plasmid, at 30° C to intermediate cell density. IPTG is then added to induce expression of the recombinant gene from the tac promoter.

Hypodermin A product accumulates in E. coli in an insoluble form, and accounts for between 0.1 and 1.0% of total E. coli protein visible by polyacrylamide gel electrophoresis (PAGE) in SDS staining with coomassie blue. The insoluble Hypodermin A product can be partially purified by differential centrifugation after the E. coli are lysed by sonication or by three passages through a cell lysis mill. Insoluble protein can be further purified by treatment with lysozyme and EDTA, followed by treatment with a nonionic detergent such as Triton X-100 (McCamen et. al., J. Biotechnology, 2:177-190 (1985)). More Specifically, lysed cultures are treated with 0.1 mg/ml egg white lysozyme in the presence of 20 mM EDTA at 25° C for 2 hours. Triton X-100 is then added to 5% v/v and the preparation stirred overnight at 4° C, or 4 hours at 25° C. The remaining insoluble protein is collected by centrifugation (30 min, 17,500 xg) resuspended 10 mM sodium phosphate, pH 7.5, extracted again with 5% Triton X-100, 20 mM EDTA stirring 4 hours at 25° C, or overnight at 4° C, collected by centrifugation. The Hypodermin A product is about 50% pure and is immunoreactive on Western blots against monoclonal and polyclonal antibodies directed against native anthestic hypodermin A.

The insoluble Hypodermin A product is solubilized by reduction and denaturation with 2% SDS and 5% B-mercaptoethanol. The insoluble hypodermin A product is also reduced and denatured by treatment with 6.75 M guanidinium-HCl, 25 mM dithiothreitol (DTT), 0.1 M Tris-HCl pH 8.0 at 50°C for 90 to 120 min. at a concentration of 0.25 mg/ml hypodermin A protein. The recombinant protein can be further purified at this point by gel filtration on Sephocryl S-300 in 5M urea. This guanidine-solubilized protein is renatured by dilution into 9 volumes of 5 M urea, 0.1 M Tris-HCl, 0.01% Tween 80, pH 8.0, and stirred for 48 hours at 4°C in an open container. Renatured samples are then dialzyed against 0.5 M urea, 50 mM Tris-HCl and concentrated by standard ultrafiltration methods to approximately 1 mg/ml.

The renatured hypodermin A protein is soluble and immuno-reactive on western blots. The preparation, however, does not have protease activity as measured by incubation in 0.1 M Tris-HCl pH8.0 with chromogenic substrates (BAEE, Sigma Chemical Co. or S-2302, KabiVitrum, Stockholm) which are cleaved under those conditions by native anthestic hypodermin A.

It is possible that the mature hypodermin A may be unstable in E. coli or lethal to the cells, because it is an active protease. In this event, synthesis of an inactive hypodermin A precursor can be achieved by the same procedure outlined above, using a synthetic linker containing sequences encoding all or part of precursor peptides up to the first unique restriction site.

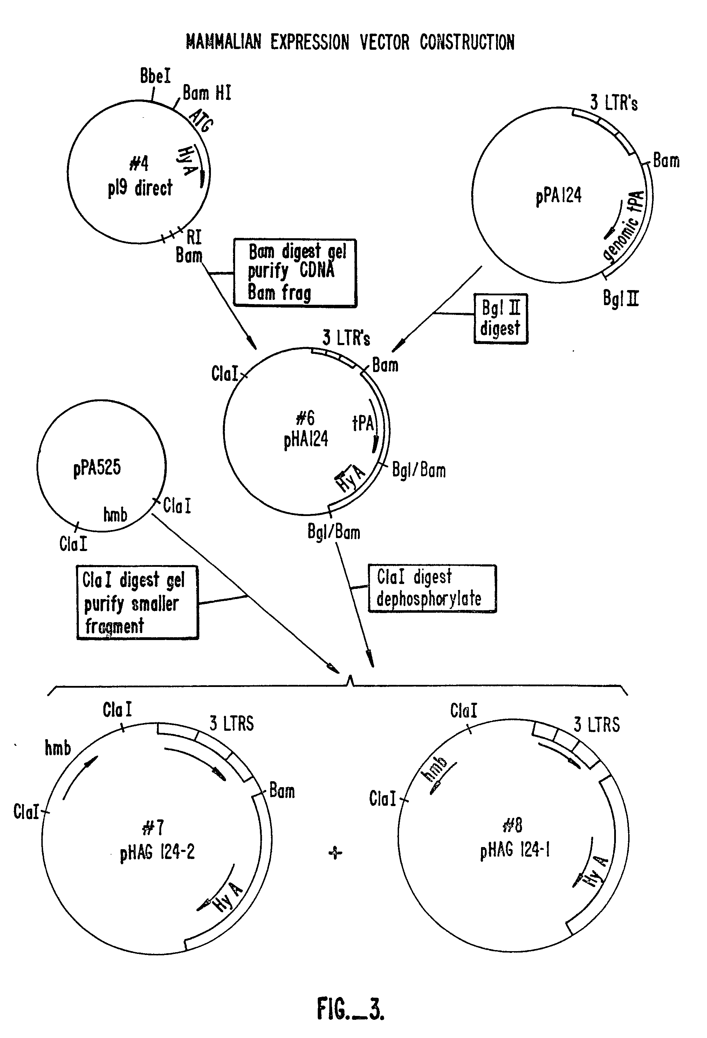

Example 4. Production of Hypodermin A in Mammalian Cells (FIG. 3)

To synthesize hypodermin A in mammalian cells, cDNA encoding mature hypodermin A is inserted into the BamHI site at the beginning of a cloned tPA gene to create a gene fusion of the sequences encoding the precursor (pre-pro) region of t-PA and the first four amino acids of mature t-PA, with mature hypodermin A.

Isolated plasmid DNA of plasmid #4 (Example 3, Fig. 2) is digested with BamHI and the cDNA fragment is gel purified. Eukaryotic expression vector pPA124 containing the tPA gene is digested with BglII, dephosphorylated with calf intestine alkaline phosphatase (CIAP), and ligated to the gel-purified cDNA fragment from plasmid #4 which carries BamHI cohesive ends which are compatible with BglII cohesive ends. E. coli is transformed with the product of this ligation, and recombinant clones are screened by restriction map for isolates with the cDNA inserted into pPA124 in the orientation indicated in plasmid #6 in Fig. 3. The N-terminal BamHI-BglII junction of plasmid #6 is verified by DNA sequencing. Plasmid DNA of #6 is prepared and digested with Cla1, dephosphorylated, and ligated to a gel-purified Cla1 fragment of pPA525 encoding hygromycin resistance. Products of this ligation are transformed into E. coli strain MH1 and plated on hygromycin plus ampicillin plates for selection of recombinant clones. Recombinant clones are screened by restriction map, and plasmids containing the hygromycin gene in each direction are recovered. These are plasmids #7 and #8 in Fig. 3.

The hypodermin A cDNA in this expression system encodes endogenous precursor and secretion signal peptides. The hypodermin A synthesized will be secreted and can be isolated from the tissue culture medium by standard protein isolation methods. Plasmid DNA is prepared from plasmids 7 and 8 and transfected into Bowes cells by the procedure of Wigler et al., Cell 16:777-785 (1979). Transfected cells are grown in Williams' Medium E(GIBCO) supplemented with 0.5% fetal calf serum.

Culture supernatants from several clones were assayed for hypodermin A protease activity using colorimetric substrate S-2302 (Kabivitrum) and were determined to have minimal proteolytic activity in this assay. Culture supernatants and cell lysates from several of the clones were analyzed by western blot using a monoclonal antibody against hypodermin A. The cell lysates contained immunoreactive material of the expected molecular weight and in an amount consistent with the expected expression level of about 10 micrograms/liter. The cell supernatants contained immunoreactive material which migrated more slowly than authentic hypodermin A and appeared as a diffuse band on the western blot. This pattern is consistent with glycosylation. Approximately 2/3 of the total immunoreactive material appeared in secreted form in the cell supernatant, the remainder in the cell lysate.

Example 5. Production of Hypodermin A in Insect Cells

Because the hypodermin A is an insect cell protein, insect cell systems are preferred for its expression. Construction of recombinant viral genomes of Autographa californica, a baculovirus routinely cultured in Spodoptera frugiperda cells, provides such a system.

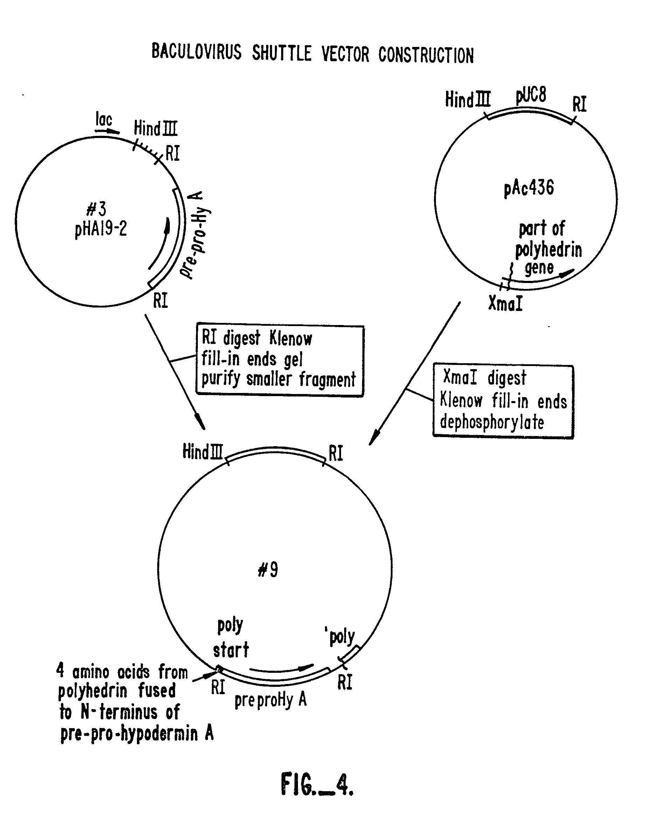

To construct the desired recombinant viral genomes, the hypodermin cDNA insert is excised from either plasmid #2 or #3 (Example 3, Fig. 2). Since the two plasmids are identical except for the orientation of the cDNA insert, either plasmid can be used. Plasmid #2 or #3 is digested with EcoRI, the ends filled in with the Klenow fragment of DNA Polymerase 1, and the cDNA fragment gel purified. DNA of plasmid pAc436 (Summers and Smith, A Manual of Methods for Baculovirus Vectors and Insect Cell Procedures, 1988) is digested with Xmal, treated with Klenow to fill in the ends, dephosphorylated, and ligated to the cDNA fragment from plasmid #2 or #3. Products of this ligation are transformed into E. coli strain MH1, and screened by restriction map for the orientation of the cDNA shown in Fig. 4. The orientation of the cDNA relative to the promoter region of pAC 436 as well as the integrity of the promoter-proximal junction are verified by DNA sequencing. The desired recombinant plasmid is plasmid #9 of Fig. 4. Plasmid #9 encodes the precursor preprohypodermin A fused at the amino terminus to four amino acids of AcNPV polyhedrin. The amino terminal sequence of the encoded protein is:

met-pro-ala-arg-asn-ser-lys-phe-val-

Amino acids 1 though 4 are the amino acids from the polyhedrin protein and amino acids 7 onwards are from the hypodermin A protein. This protein contains the necessary signal sequence for secretion of hypodermin A.

DNA of plasmid #9 is isolated and used to cotransfect Spodoptera frugiperda (Sf9) cells along with purified Autographa californica (AcNPV) viral DNA by method 1 described in Summers and Smith above. The cDNA-containing region of plasmid #9 is integrated into AcNPV genome by homologous recombination, yielding recombinant viral genomes. Recombinant viral clones are selected and purified by visual screening by the method of Summer and Smith, above. Monolayers of Sf9 cells in T-25 flasks are infected with purified recombinant clones, grown for 5 days, and the cell lysates and supernatants analyzed by assay of hypodermin A enzymatic activity using colorimetric substrate S-2302, and by western blot for immunoreactive material.

The cell supernatants have significant amounts of enzymatically active material, consistent with expression of correctly folded and secreted hypodermin A. Western blots show immunoreactive material in both the cell lysates and supernatants, in approximately equal total amounts. The supernatant material runs as a single band, while the lysate material appears as at least four distinct species, consistent with various stages of cleavage and processing prior to secretion. Total expression levels are in the range of about 10mgs/liter.

Alternatively, cDNA encoding the mature form of hypodermin A is purified from plasmid #4 (Example 3, Fig. 2) and similarly cloned into baculovirus shuttle vectors from Summers and Smith. Recombinant virus are similarly purified from co-transfection supernatants, and analyzed for immunoreactive material and enzymatic activity.

Western blots show that the great majority of immunoreactive material is in the cell lysates, as expected. The small amount present in the cell supernatants could be attributed to cell lysis late in infection.

## Example 6. Use of the Hypodermin A signal sequence to achieve secretion of heterologous proteins in insect cells

The control sequences of the baculovirus polyhedrin gene allow high level expression of heterologous genes in insect cells, but because polyhedrin is not a secreted protein, its control sequences do not enable the secretion of the heterologous gene product. Hypodermin A is a protein naturally synthesized and secreted in large amounts by the insect Hypoderma lineatum. The secretion signal sequences encoded by the hypodermin A gene can be used to effectively enable the secretion of heterologous proteins in insect cells. Because hypodermin A is an insect protein and is naturally synthesized and secreted in large amounts, it offers a preferred method for secretion of heterologous gene products in insect cells using the baculovirus or other insect cell expression system.

It is possible to fuse to the signal sequence portion of the hypodermin A gene other genes that one wishes to express as secreted proteins in the baculovirus system. It is probable that the hypodermin A signal peptide, being an insect cell sequence, is more efficiently recognized by Sf9 cells than a signal peptide encoded by non-insect (e.g. mammalian) genome. Thus, replacing the signal peptide of tPA, for example, with the signal peptide of hypodermin A might increase the overall expression of tPA in a baculovirus system, and/or improve the specific activity of the expressed, secreted protein.

An example of how such a fusion might be constructed follows. Plasmid #2, containing prepro-hypodermin A is digested with BamHI and BsmI (and Sst I to destroy the SamII fragment). Into the large fragment of this digested plasmid #2 is ligated synthetic DNA containing a BamHI site, an ATG codon, DNA encoding the prepro-hypodermin A signal peptide (see Table IV) and the first 8 amino acids of the hypodermin A propeptide (ala val val pro leu gly met leu) up to and including the BsmI site. The resulting plasmid (plasmid 10) encodes prepro-hypodermin A, including the membrane signal sequence, bounded by unique BamHI and EcoRI sites.

Plasmid 10 is cleaved with BsmI, a unique site, and the desired gene sequence is ligated into plasmid 10 at the BsmI site. Before ligation the plasmid can be treated with mung bean nuclease if a non-fused protein is desired. Products of the ligation, after transformation, would be screened by restriction mapping and/or DNA sequencing to determine a junction in a correct reading frame. Linkers ligated to the gene fragment of interest and to the ends of the vector could be used to facilitate construction. The desired plasmid product, plasmid 11, is cleaved with BamHI and EcoRI and the cDNA-containing fragment ligated to BamHI - EcoRI digested pVL 1393, a baculovirus shuttle vector obtained from Dr. Max Summers, Department of Agriculture Biotechnology, Texas A&M University.

For more general use, a BsmI-cleaved sample of plasmid 10 can be treated with mung bean nuclease, ligated to a short synthetic DNA fragment encoding a series of restriction sites, and the resultant clones screened by DNA sequencing for those that have acquired a polylinker sequence at the appropriate position (i.e., just downstream of the signal peptide processing site). Heterologous genes could then be inserted into the appropriate site in the polylinker so that the gene will be expressed in the baculovirus system fused to the hypodermin A signal peptide prior to cleavage.

Alternatively, DNA could be chemically synthesized that contains sequences encoding the hypodermin signal, and the first four amino acids of the propeptide followed by restriction sites for insertion of heterologous genes. This synthetic DNA can be inserted into a vector such as pUC19 to yield pUC19-hypodermin A signal peptide. Once the desired heterologous gene has been cloned into an appropriate site downstream from the signal peptide, the entire fragment encodoing the signal and gene could be moved into a baculovirus shuttle vector such as pVL1393 for expression.

The amino acid sequence of the hypodermin A membrane secretion signal peptide is:

met-leu-lys-phe-val-ile-leu-leu-cys-ser-ile-ala-tyr-val-phe-gly-

The amino acid sequence of the hypodermin B membrane secretion signal peptide is met-leu-lys-phe-val-ile-leu-val-cys-ser-val-ala-cys-val-phe-gly-.

These sequences were determined by comparing the DNA sequences of cDNA clones encoding hypodermin A and hypodermin B (Tables II and III)

Example 7. Immunoprotection using native hypodermin

Yearling Hereford heifers were purchased at an age of between 4-5 months. At about 9 months of age the heifers were provided with immunoprotection from H. lineatum larva by a series of four injections. The vaccines were comprised of 0.5 mg of either hypodermin A (fraction 4, Fig. 1) or a mixture of hypoderm B and C (fraction 7, Fig. 1) in 1 ml of 10 mM Tris-HCl buffer 1:1, with complete or incomplete Freund's adjuvant. The injection protocol involves repeated injections every two weeks for a total of four injections over a six-week period. The total antigen is 2 mg. The first two injections use complete Freund's adjuvant. The last two injections use incomplete adjuvant. In the studies conducted, it was demonstrated under laboratory conditions that larval survival, as measured by development of warbles, is significantly reduced by the treatment of cattle with these vaccines.

In a first set of trials, animal experimental groups were set up and challenged with between 30 and 54 hatched larvae in a laboratory infestation. The animals were then released to pasture and were potentially further exposed to the endemic population of cattle grub flies. Larval mortality in those animals vaccinated with hypodermin A was 89.9% compared to 60.9% for previously infested nonvaccinated animals and 35% for naive control animals. The data suggest that a high level of protection from hypodermosis can be elicited when naive animals are immunized with purified hypodermin A from the first-instar of Hypoderma lineatum.

In a second set of trials, hypodermin A-vaccinated, B-C-vaccinated, and control heifers were infested, respectively, with an average of 145.3, 125.2, and 116.6 larvae per animal. This infectious dose represents an unnaturally high number of larvae. Mortality of larvae, as they progressed toward the back, was 62.73% for hypodermin A-vaccinated animals, 61.01% for B-C-vaccinated animals, and 44.17% for control animals. Although this level of mortality to the back is comparable to the levels found in previously infested, unvaccinated animals having naturally acquired resistance, the percent mortality was lower than the mortality rates of our previous studies. It is believed that the low mortality is due to the unrealistically high numbers of larvae used to challenge the animals. Total mortality in which the larvae died at some point in development to the back, or in the back was 98.07% for hypodermin A-vaccinated animals, 96.33% for B-C-vaccinated animals, and 88.61% for control animals. Total pupal yield was 24 for the hypodermin A-vaccinated animals, 49 for B-C-vaccinated animals, and 103 for control animals. Thus, it was demonstrated that naive animals can be artificially induced through vaccination to levels of resistance that equal or exceed the level of resistance found in those animals having previous infestations.

13

EP 0 326 419 A2

Table I
cDNA Sequence for Hypodermin A As Found in ATTC Deposit # 67612

```
                                                    -28
                                                    lys   phe   val   ile   leu
                                        GAATC   C   AAG   TTT   GTT   ATT   TTA

                          -20                                         -10
leu   cys   ser   ile   ala   tyr   val   phe   gly | ala   val   val   pro   leu   gly
TTG   TGC   AGT   ATT   GCC   TAT   GTT   TTC   GGT  GCC   GTC   GTA   CCA   TTG   GGA

                                                      | 1
met   leu   ser   gln   ser   asp   gly   arg   ile   val   gly   gly   val   glu   ser
ATG   CTT   TCT   CAA   TCG   GAT   GGA   CGT   ATT   GTT   GGT   GGT   GTT   GAA   TCC

            10                                                      20
lys   ile   glu   asp   phe   pro   trp   gln   ile   ser   leu   gln   arg   asp   gly
AAG   ATT   GAA   GAT   TTT   CCT   TGG   CAA   ATA   TCC   CTC   CAA   CGC   GAT   GGT

                                          30
arg   his   tyr   cys   gly   gly   ser   ile   tyr   ser   lys   asn   val   ile   ile
CGT   CAT   TAC   TGC   GGA   GGT   TCC   ATC   TAC   AGC   AAA   AAT   GTA   ATT   ATC

            40                                                      50
thr   ala   ala   his   cys   leu   arg   asn   val   val   ala   glu   glu   leu   arg
ACT   GCC   GCC   CAC   TGC   CTA   AGA   AAT   GTT   GTA   GCA   GAA   GAA   CTC   AGA

                                          60
val   arg   val   gly   ser   ser   tyr   trp   glu   his   gly   gly   ser   leu   arg
GTT   CGC   GTT   GGT   TCC   TCT   TAT   TGG   GAG   CAT   GGA   GGC   TCT   TTG   CGT

            70                                                      80
asn   ile   ser   lys   phe   gln   ile   his   glu   ser   tyr   val   glu   pro   thr
AAT   ATA   TCC   AAA   TTT   CAA   ATA   CAC   GAA   TCC   TAC   GTC   GAA   CCA   ACT

                                          90
lys   glu   tyr   asp   val   ala   leu   leu   lys   leu   asp   ser   asp   leu   ser
AAA   GAG   TAC   GAT   GTA   GCT   CTT   CTA   AAA   TTG   GAT   TCT   GAT   TTA   TCA

            100                                                     110
phe   asn   ser   thr   ile   lys   ala   ile   glu   leu   thr   asn   glu   ile   pro
TTC   AAT   TCA   ACG   ATT   AAA   GCT   ATT   GAA   CTG   ACC   AAC   GAA   ATA   CCT

                                          120
pro   glu   tyr   ala   asp   ala   ile   val   ser   gly   trp   gly   glu   thr   leu
CCC   GAA   TAT   GCT   GAC   GCT   ATT   GTT   TCT   GGT   TGG   GGT   GAA   ACC   CTT

            130                                                     140
val   pro   pro   pro   gly   ile   pro   asp   gln   leu   arg   ser   val   asp   val
GTT   CCT   CCT   CCT   GGT   ATT   CCT   GAT   CAA   TTA   AGA   TCC   GTT   GAT   GTT

                                          150
lys   ile   ile   his   arg   glu   lys   cys   ala   ser   arg   asn   phe   gly   tyr
AAA   ATC   ATT   CAT   AGA   GAA   AAG   TGT   GCA   TCA   AGA   AAT   TTC   GGT   TAC
```
--------------------------------------------------------------------------------
Key: aa -28; third amino acid of hypodermin A signal 'pre' peptide
     aa -14; start of hypodermin A propeptide
     aa 1; start of mature hypodermin A

14

```
                160                                                    170
gly  ser  asn  ile  lys  ala  ser  met  ile  cys  ala  tyr  ala  ile  gly
GGT  TCT  AAT  ATT  AAA  GCC  AGT  ATG  ATC  TGT  GCT  TAT  GCT  ATC  GGC


                                        180
lys  asp  ser  cys  gln  gly  asp  ser  gly  gly  pro  leu  val  val  asn
AAA  GAC  TCC  TGC  CAA  GGT  GAT  TCT  GGT  GGT  CCT  TTG  GTT  GTC  AAT


                190                                              200
asn  leu  leu  val  gly  val  val  ser  trp  gly  ile  asp  cys  ala  arg
AAC  CTT  TTA  GTT  GGT  GTT  GTC  TCA  TGG  GGT  ATA  GAT  TGC  GCT  AGG


                                        210
pro  ser  tyr  pro  gly  val  tyr  val  asp  val  ser  his  val  arg  ser
CCC  TCA  TAC  CCA  GGT  GTA  TAC  GTC  GAT  GTG  TCC  CAC  GTT  CGT  TCT


                220                          226
trp  ile  val  ser  asn  ala  glu  ser  ile  STOP
TGG  ATC  GTC  AGT  AAT  GCA  GAA  TCT  ATT  TAA   GCCCTGATTTTGACTTGTAAT
```

TGTTTTTAATAAAAGAGTATATCAAAATAATAACTGAAATTGATAAAGCTCAAAAAAAAA

AAAAAAAGGAATTC

Translated Mol. Weight = 30453.86

---

Key: aa -28; third amino acid of hypodermin A signal 'pre' peptide
     aa -14; start of hypodermin A propeptide
     aa 1; start of mature hypodermin A

15

## Table II
### cDNA Sequence for Hypodermin B As Found in ATTCC Deposit # 67613

```
                                                        GAATTC   CTAT

              -30                                        -20
              met leu lys phe val ile leu val cys ser    val ala cys
ATCAGT        ATG TTG AAG TTC GTT ATT TTA GTG TGC AGT    GTC GCC TGC


                                        -10
val phe gly ala val val pro gly gly met leu pro gln leu asp
GTA TTT GGT GCC GTC GTA CCT GGG GGA ATG CTT CCT CAA CTA GAT


         1                                          10
gly arg ile val gly gly phe glu ala asp ile glu asp phe pro
GGG CGC ATT GTT GGT GGC TTT GAG GCC GAT ATC GAA GAT TTT CCT


                            20
trp gln val ser ile gln arg gly gly tyr his phe cys gly gly
TGG CAA GTT TCC ATC CAA CGT GGA GGT TAT CAT TTC TGC GGA GGA


    30                                          40
ser ile tyr ser pro glu ile ile val thr ala ala his cys leu
TCC ATC TAC AGC CCG GAG ATC ATT GTC ACT GCT GCC CAT TGC TTG


                        50
glu lys ile asp ala ser gln leu arg val arg val gly gly ser
GAA AAA ATT GAT GCA TCC CAA CTT AGA GTT CGC GTT GGT GGC TCC


    60                                          70
tyr trp asp glu glu gly ser leu leu thr val ser asn phe lys
TAT TGG GAC GAA GAA GGT TCC TTG CTT ACC GTA TCC AAT TTT AAA


                        80
ile his glu lys tyr asp ala met ile met trp tyr asp val ala
ATA CAC GAA AAG TAC GAC GCA ATG ATT ATG TGG TAC GAT GTA GCT


    90                                          100
leu leu lys leu ser ser lys leu thr tyr gly ala thr val lys
CTT TTA AAA TTA AGT TCA AAA TTA ACA TAT GGT GCC ACG GTA AAA


                        110
asn ile glu leu ala lys glu thr pro pro asp asn ala asp ala
AAT ATC GAG TTG GCT AAA GAG ACT CCT CCC GAC AAC GCT GAT GCT


    120                                         130
val val ser gly trp gly thr ile tyr glu asn tyr pro tyr met
GTT GTT TCT GGT TGG GGT ACA ATA TAT GAA AAT TAT CCA TAT ATG


                        140
pro val gln leu arg ser val asp val lys ile val ser arg glu
CCA GTA CAA TTA AGA TCC GTT GAT GTT AAA ATC GTT AGC CGG GAA
```

Key: aa -30; start of hypodermin B signal 'pre' peptide
     aa -14; start of hypodermin B propeptide
     aa 1; start of mature hypodermin B

```
        150                                             160
val cys arg ser asp glu tyr gly tyr gly asn ala ile gly pro
GTG TGT AGG TCA GAC GAA TAC GGT TAT GGT AAT GCT ATC GGA CCC

                                170
thr met ile cys ala tyr ala val gly lys asp ala cys gln gly
ACT ATG ATC TGT GCT TAT GCT GTA GGC AAA GAC GCC TGC CAA GGT

        180                                             190
asp ser gly gly pro leu val val gly glu ala leu val gly val
GAT TCT GGT GGT CCC TTG GTT GTC GGA GAA GCT TTA GTT GGT GTT

                                200
val ser trp gly glu gly cys ala tyr pro gly phe pro gly val
GTC TCA TGG GGT GAG GGA TGC GCG TAC CCT GGA TTC CCG GGT GTC

        210                                             220
tyr thr asp val ser val val arg ser trp ile thr glu asn ala
TAC ACC GAT GTG TCT GTT GTT CGC TCC TGG ATT ACT GAA AAT GCA

        226
lys ser phe STOP
AAA TCT TTT TAA  AACTTGATATCAATTTGTAACTATTTTTAATAAAAAGAGCTCTTT

AAAACAAAAAAAAAAAAAAGGAATTC
```

Translated Mol. Weight = 27715.32

--------------------------------------------------------------------------------
Key: aa -30; start of hypodermin B signal 'pre' peptide
     aa -14; start of hypodermin B propeptide
     aa 1; start of mature hypodermin B

Table III
Nucleic Acid Sequence of Synthetic
Oligonucleotides COD 872 and COD 873

```
COD 872 (42 nuleotides in length)
5'                                                        3'
   3    6    9   12   15   18   21   24   27   30   33   36   39   42
  CGG  ATC  CAT  GAT  TGT  TGG  TGG  TGT  TGA  ATC  CAA  GAT  TGA  AGA


COD 873 (46 nucleotides in length)
5'                                                              3'
   3    6    9   12   15   18   21   24   27   30   33   36   39   42   45   46
  TCT  TCA  ATC  TTG  GAT  TCA  ACA  CCA  CCA  ACA  ATC  ATG  GAT  CCG  GCG  C
```

## Table IV
### Synthetic DNA for Baculovirus Shuttle Vector Encoding a BamHI Site, an ATG Codon, Preprohypodermin A Signal Peptide, the First Eight Amino Acids of the Hypodermin A Propeptide and a BsmI Site

| BamHI | | met | leu | lys | phe | val | ile | leu |
|---|---|---|---|---|---|---|---|---|
| GA | TCC | ATG | CTC | AAG | TTT | GTT | ATT | TTA |
|  | G | TAC | GAG | TTC | AAA | CAA | TAA | AAT |

| leu | lys | ser | ile | ala | tyr | val | phe | gly |
|---|---|---|---|---|---|---|---|---|
| TTG | TGC | AGT | ATT | GCC | TAT | GTT | TTC | GGT |
| AAC | ACG | TCA | TAA | CGG | ATA | CAA | AAC | CCA |

| ala | val | val | pro | leu | gly | met | leu |
|---|---|---|---|---|---|---|---|
| GCC | GTC | GTA | CCA | TTG | GGA | ATG | CT |
| CGG | CAG | CAT | GGA | AAC | CCT | TAC | |
|  |  |  |  |  |  | BsmI | |

## Claims

1. A plasmid comprising a cDNA sequence coding for a protein from the group consisting of hypodermins A, B, and C.

2. A plasmid of claim 1 wherein the plasmid facilitates expression of the cDNA sequence by a suitable host.

3. A suitable host containing a plasmid comprising a DNA sequence coding for a protein from the group consisting of hypodermins A, B, and C.

4. A suitable host according to claim 3 wherein the plasmid facilitates expression of the DNA sequence coding for a protein selected from the group consisting of hypodermins A, B, and C.

5. A suitable host according to claim 2 or 3 wherein the host is a bacterium, a yeast or a eukaryote cell.

6. A suitable host according to claim 4 wherein the protein encoded by the DNA sequence is hypodermin A.

7. A suitable host according to claim 4 wherein the host is a bacterium, yeast or a eukaryote cell.

8. A vaccine for protection of animals from hypoderma species comprising injectables of non hypoderma origins and immunologically pure Hypoderma protein selected from the group consisting of:
   a. hypodermin A;

b. hypodermin B;

c. substantially pure hypodermin C;

d. a mixture of hypodermin B and hypodermin C;

e. a mixture of hypodermin A and hypodermin B; and

f. a mixture of substantially pure hypodermin A, hypodermin B, hypodermin C, and immunogenic equivalents thereof.

9. The vaccine according to claim 8 wherein the Hypoderma species is lineatum or bovis.

10. The vaccine according to claim 8 containing essentially pure hypodermin A and trace amounts of hypodermin B and hypodermin C.

11. The vaccine according to claim 9 wherein the hypodermin A is substantially pure.

12. The vaccine according to claim 9 wherein the hypodermin B is substantially pure.

13. The vaccine according to claim 9 wherein the hypodermin C is substantially pure.

14. The vaccine according to claim 9 comprising a mixture of hypodermin B and hypodermin C, without trace amounts of hypodermin A.

15. The vaccine according to claim 14 wherein the mixture of hypodermin B and hypodermin C is substantially pure.

16. A method for protecting animals from infection of Hypoderma species which comprises the vaccination of the animals with a vaccine consisting of injectables of non-hypoderma origins and immunologically pure Hypoderma protein selected from the group consisting of:

a. hypodermin A;

b. hypodermin B;

c. substantially pure hypodermin C;

d. a mixture of hypodermin B and hypodermin C;

e. a mixture of hypodermin A and hypodermin B;

and

f. a mixture of substantially pure hypodermin A, hypodermin B, hypodermin C.

17. The method according to claim 16 wherein the Hypoderma protein comprises hypodermin A.

18. The method according to claim 17 wherein the hypodermin A is substantially pure.

19. The method according to claim 16 wherein the hypodermin B is substantially pure.

20. The method according to claim 16 wherein the hypodermin C is substantially pure.

21. The method according to claim 16 comprising a mixture of hypodermin B and hypodermin C, without trace amounts of hypodermin A.

22. The method according to claim 21 wherein the mixture of hypodermin B and hypodermin C is substantially pure.

23. The method of claim 16 wherein the Hypoderma species is lineatum.

24. A vaccine comprising a protein derived from a plasmid of claim 1.

25. A vaccine of claim 8 comprising at least one epitope of Hypodermin A, B or C.

26. A plasmid comprising a nucleotide sequence encoding the secretion signal peptide of hypodermin A or B.

27. A plasmid comprising a nucleic acid sequence encoding a secretion signal peptide, wherein the peptide is:

met-lys-lys-phe-val-ile-leu-leu-cys-ser-ile-ala-tyr-val-phe-gly-.

28. A plasmid comprising a nucleic acid sequence encoding a secretion signal peptide, wherein the peptide is:

met-leu-lys-phe-val-ile-leu-val-cys-ser-val-ala-cys-val-phe-gly-.

FIG._I.

EP 0 326 419 A2

EXAMPLE 3. E. COLI EXPRESSION PLASMID CONSTRUCTION SUMMARY

FIG._2.

FIG._3.

## BACULOVIRUS SHUTTLE VECTOR CONSTRUCTION

lac Hind III RI

#3 pHAl9-2 pre-pro-Hy A RI

Hind III pUC8 RI

pAc436 part of polyhedrin gene XmaI

RI digest Klenow fill-in ends gel purify smaller fragment

XmaI digest Klenow fill-in ends dephosphorylate

Hind III RI

#9

poly start RI preproHy A poly RI

4 amino acids from polyhedrin fused to N-terminus of pre-pro-hypodermin A

FIG._4.